(19) Europäisches Patentamt
European Patent Office
Office européen des brevets

(11) **EP 3 022 311 B1**

(12) **EUROPÄISCHE PATENTSCHRIFT**

(45) Veröffentlichungstag und Bekanntmachung des Hinweises auf die Patenterteilung:
**18.09.2019 Patentblatt 2019/38**

(51) Int Cl.:
**C12N 11/04** *(2006.01)*  **C12P 19/24** *(2006.01)*
**C12P 19/12** *(2006.01)*  **C12N 11/02** *(2006.01)*

(21) Anmeldenummer: **14739832.5**

(22) Anmeldetag: **17.07.2014**

(86) Internationale Anmeldenummer:
**PCT/EP2014/065358**

(87) Internationale Veröffentlichungsnummer:
**WO 2015/007833 (22.01.2015 Gazette 2015/03)**

(54) **OPTIMIERTES VERFAHREN ZUR HERSTELLUNG EINER ISOMALTULOSE-HALTIGEN ZUSAMMENSETZUNG**

OPTIMISED METHOD FOR PRODUCING A COMPOSITION CONTAINING ISOMALTULOSE

PROCÉDÉ D'OPTIMISATION POUR LA PRODUCTION D'UNE COMPOSITION CONTENANT DE L'ISOMALTULOSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2013 DE 102013011977**

(43) Veröffentlichungstag der Anmeldung:
**25.05.2016 Patentblatt 2016/21**

(73) Patentinhaber: **Südzucker AG**
**68165 Mannheim (DE)**

(72) Erfinder:
• **WACH, Wolfgang**
**67549 Worms (DE)**
• **ROSE, Thomas**
**67549 Worms (DE)**

(74) Vertreter: **Schrell, Andreas et al**
**Gleiss Große Schrell und Partner mbB**
**Patentanwälte Rechtsanwälte**
**Leitzstrasse 45**
**70469 Stuttgart (DE)**

(56) Entgegenhaltungen:
EP-A1- 0 625 578    EP-A2- 0 049 801
EP-A2- 0 483 755    WO-A1-97/44478

• DE OLIVA-NETO P ET AL: "Isomaltulose production from sucrose by Protaminobacter rubrum immobilized in calcium alginate", BIORESOURCE TECHNOLOGY, ELSEVIER BV, GB, Bd. 100, Nr. 18, 1. September 2009 (2009-09-01), Seiten 4252-4256, XP026130078, ISSN: 0960-8524, DOI: 10.1016/J.BIORTECH.2009.03.060 [gefunden am 2009-05-01]
• KAWAGUTI H Y ET AL: "Production of isomaltulose using Erwinia sp. D12 cells: Culture medium optimization and cell immobilization in alginate", BIOCHEMICAL ENGINEERING JOURNAL, ELSEVIER, AMSTERDAM, NL, Bd. 29, Nr. 3, 15. April 2006 (2006-04-15) , Seiten 270-277, XP028034513, ISSN: 1369-703X, DOI: 10.1016/J.BEJ.2006.01.006 [gefunden am 2006-04-15]
• KRASTANOV A ET AL: "Conversion of sucrose into palatinose in a batch and continuous processes by immobilized Serratia plymuthica cells", ENZYME AND MICROBIAL TECHNOLOGY, STONEHAM, MA, US, Bd. 39, Nr. 6, 3. Oktober 2006 (2006-10-03), Seiten 1306-1312, XP027948909, ISSN: 0141-0229 [gefunden am 2006-10-03]

**Beschreibung**

Die vorliegende Erfindung betrifft ein Verfahren zur Herstellung einer Isomaltulose-haltigen Zusammensetzung aus einem Saccharose-haltigen Substrat.

Bekanntermaßen ist Isomaltulose (Palatinose, 6-O-α-D-Glucopyranosyl-D-fructose) in einem biotechnologischen Prozess durch enzymatische Isomerisierung aus Saccharose herstellbar. Isomaltulose ist ein physiologisch wertvoller Zucker, der als Saccharoseaustauschstoff in Lebensmitteln und verwandten Produkten zunehmend an Bedeutung gewinnt. Isomaltulose ist akariogen und besitzt einen niedrigen glykämischen Index bei im Wesentlichen demselben Energiegehalt wie Saccharose. Isomaltulose ist seit 2005 zur Verwendung in Lebensmitteln zugelassen. Isomaltulose ist außerdem Ausgangsstoff für die Herstellung des Zuckeralkohols Isomalt, eines razemischen Gemisches aus 1,6-GPS (6-O-α-D-Glucopyranosyl-D-sorbit) und 1,1-GPM (1-O-α-D-Glucopyranosyl-D-mannit) sowie Abwandlungen davon, besonders 1,6-GPS- oder 1,1-GPM-angereicherter Gemische.

In den bekannten biotechnologischen Systemen wird Saccharose enzymatisch nicht vollständig zu Isomaltulose isomerisiert. Es entstehen vielmehr weitere Isomerisierungsprodukte und Nebenprodukte. Ein wesentliches weiteres Isomerisierungsprodukt ist Trehalulose.

Trehalulose (1-O-α-D-Glucopyranosyl-D-fructose) als ein weiteres wesentliches Isomer der Saccharose findet sich in der Natur, beispielsweise in Honig, und ist ebenso wie Isomaltulose nicht kariogen.

Beide Isomere Isomaltulose und Trehalulose sind zum Beispiel aus der EP 1 424 074 A1 als Heterodisaccharide bekannt, die im Vergleich zu Saccharose im Dünndarm menschlicher und tierischer Konsumenten eine reduzierte Hydrolyserate aufweisen und sich daher als vorteilhafte Nahrungsmittelkomponenten darstellen, insbesondere als solche, die den Blutzuckerspiegel verbessert kontrollierbar halten.

Großtechnisch werden Isomaltulose und Trehalulose durch enzymatische Umlagerung unter Verwendung von immobilisierten Bakterienzellen oder Fragmenten davon hergestellt. Dabei wird die zwischen den Monosaccharideinheiten des Disaccharids Saccharose bestehende a1-a2-glycosidische Bindung zu einer a1-a6-Bindung bei Isomaltulose bzw. einer α1-α1-Bindung bei Trehalulose isomerisiert. Diese Umlagerung von Saccharose zu den beiden akariogenen Disacchariden erfolgt unter Katalyse des bakteriellen Enzyms Saccharoseisomerase (E.C. 5.4.99.11, synonym: Sucrosemutase, Saccharosemutase, Sucroseisomerase, Isomaltulosesynthase). Mikroorganismen, die Saccharoseisomeraseaktivität besitzen und in biotechnologischen Prozessen eingesetzt werden können, sind insbesondere Protaminobacter rubrum, Erwinia rhapontici, Pseudomonas mesoacidophila, Pantoea dispersa und Serratia plymuthica. Je nach verwendetem Organismus und Reaktionsbedingungen ergibt sich bei dieser Reaktion ein Produktgemisch, das neben den erwünschten akariogenen Disacchariden Isomaltulose und Trehalulose in unterschiedlichen Mengenverhältnissen auch gewisse Anteile an ggf. unerwünschten Monosacchariden, zum Beispiel Glucose und/oder Fructose, sowie Oligomere enthalten kann.

Die EP 0 483 755 B1 offenbart Verfahren zur Herstellung von Trehalulose- und Isomaltulose-haltigen Zusammensetzungen, wobei zu diesem Zweck Pseudomonas mesoacidophila MX-45 (FERM-BP 3619) oder Agrobacterium radiobacter MX-232 (FERM-BP 3620) eingesetzt wird, um ein vorwiegend Trehalulose-haltiges Produkt zu erhalten.

Der Einsatz von enzymatischen Aktivitäten aus Pseudomonas putida, Thermus ruber, Thermus aquatica oder Pimelobacter zur Herstellung vorwiegend Trehalulose-haltiger Zusammensetzungen aus Saccharose-haltigen Lösungen ist aus der EP 0 794 259 B1 bekannt.

Die EP 0 091 063 A2 und EP 0 625 578 A1 offenbaren Verfahren zur Herstellung von Isomaltulose unter Verwendung immobilisierter bakterieller Zellen, insbesondere Protaminobacter rubrum (CBS 574.77). Die offenbarten Verfahren gehen von einer Saccharose-haltigen Lösung aus, die unter Einsatz immobilisierter Zellen in ein vorwiegend Isomaltulose- aber auch Trehalulose-haltiges Produkt überführt wird.

Die Oliva-Neto et al. ("Isomaltulose production from sucrose by Protaminobacter rubrum immobilized in calcium alginate", Bioresource Technology, Vol. 100, No. 18, Seiten 4252-4256) offenbart die Herstellung von Isomaltulose aus Saccharose mittels Protaminobacter rubrum, wobei Zellen aus Protaminobacter rubrum in Calcium-Alginat mit Glutaraldehyd und Polyethylenimin immobilisiert werden.

Kawaguti et al. ("Production of Isomaltulose using Erwinia sp. D12 cells: Culture medium optimization and cell immobilization in alginate", Biochemical Engineering Journal, Bd. 29, Nr. 3, 2006, Seiten 270-277) offenbart ein Verfahren zur Herstellung von Isomaltulose mit in Natrium-Alginat immobilisierten Zellen.

Die offenbarten Verfahren zeichnen sich allesamt durch eine limitierte Aktivität der Saccharoseisomerase aus, wobei insbesondere für eine großtechnische Herstellung der Isomaltulose, Trehalulose oder von beiden eine verbesserte Verfahrenseffizienz wünschenswert ist, insbesondere eine schnellere Umsatzgeschwindigkeit und/oder erhöhte Umsatzrate und/oder Ausbeute.

Der vorliegenden Erfindung liegt daher das technische Problem zugrunde, die vorgenannten Nachteile zu überwinden, insbesondere ein Verfahren bereitzustellen, im Rahmen dessen ein gegenüber dem Stand der Technik verbesserter Umsatz von Saccharose zu einem Isomaltulose- und/oder Trehalulose-haltigen Produkt erzielt wird. Insbesondere ist es ein Ziel der vorliegenden Erfindung, eine erhöhte Umsatzgeschwindigkeit und/oder eine erhöhte Umsatzrate und/oder Ausbeute bereitzustellen.

Die vorliegende Erfindung löst das ihr zugrundeliegende technische Problem durch die Bereitstellung der Lehre gemäß des unabhängigen Anspruchs.

Insbesondere stellt die vorliegende Erfindung folgende Lehren bereit.

Die vorliegende Erfindung betrifft insbesondere ein erfindungsgemäßes Verfahren zur Herstellung einer Isomaltulose-haltigen Zusammensetzung aus einem Saccharose-haltigen Substrat, umfassend die folgenden Verfahrensschritte:

a) Inkontaktbringen des Saccharose-haltigen Substrates mit einer partikulären Träger-immobilisierten Saccharoseisomerase-Biomasse und

b) Erhalten einer Isomaltulose-haltigen Zusammensetzung,

dadurch gekennzeichnet, dass die Träger-immobilisierte Saccharoseisomerase-Biomasse einen Medianwert der Partikelgrößenverteilung bezogen auf die Massen- oder Volumenverteilung (d(0,5)-Wert, bezogen auf in trockener Form gemessene Partikel) von 370 bis 550 $\mu$m aufweist und der Träger ein Alginat- oder Polyvinylalkohol-Träger ist.

Die vorliegende Erfindung betrifft insbesondere ein erfindungsgemäßes Verfahren, wobei die partikuläre Träger-immobilisierte Saccharoseisomerase-Biomasse kugelförmig ist.

In besonders bevorzugter Ausführungsform kann die partikuläre Träger-immobilisierte Saccharoseisomerase-Biomasse die Form eines Würfels, einer Platte, eines Partikels, einer Faser, insbesondere einer Hohlfaser, einer Kugel, einer Hohlkugel oder eines LentiKats® aufweisen.

Die vorliegende Erfindung betrifft insbesondere ein erfindungsgemäßes Verfahren, wobei das Gewichtsverhältnis von Saccharoseisomerase-Biomasse zu Träger 10 bis 6, bevorzugt 7, Teile Saccharoseisomere-Biomasse zu 6 bis 2, bevorzugt 3, Teile Träger (jeweils Trockengewicht) beträgt.

In besonders bevorzugter Ausführungsform beträgt das Gewichtsverhältnis von Saccharoseisomerase-Biomasse zu Träger 0,2 bis 0,8, insbesondere 0,3 bis 0,7 oder bevorzugt 0,4 bis 0,6.

Die vorliegende Erfindung betrifft insbesondere ein erfindungsgemäßes Verfahren, wobei die Isomaltulose-haltige Zusammensetzung Trehalulose enthält.

Die vorliegende Erfindung betrifft insbesondere ein erfindungsgemäßes Verfahren, wobei die Saccharoseisomerase-Biomasse durch Adsorption, Bindung, insbesondere kovalente Bindung, Quervernetzung, Verkapselung oder Einschlussimmobilisierung Träger-immobilisiert ist.

Die vorliegende Erfindung betrifft ein erfindungsgemäßes Verfahren, wobei der Träger ein Alginat- oder Polyvinylalkohol-Träger ist, insbesondere ein Natriumalginat-Träger ist.

Die vorliegende Erfindung betrifft insbesondere ein erfindungsgemäßes Verfahren, wobei die Saccharoseisomerase-Biomasse eine Saccharoseisomerase, eine Mikroorganismenzelle mit Saccharoseisomerase-Aktivität oder ein Zellextrakt mit Saccharoseisomerase-Aktivität ist.

Die vorliegende Erfindung betrifft insbesondere ein erfindungsgemäßes Verfahren, wobei die Saccharoseisomerase-Biomasse aus Mikroorganismen der Gattungen Escherichia, Salmonella, Serratia, Erwinia, Enterobacter, Klebsiella, Raoultella, Pectobacterium, Pseudomonas, Azotobacter, Pantoea, Leucanea, Protaminobacter oder Bacillus sp. stammt.

Die vorliegende Erfindung betrifft insbesondere ein erfindungsgemäßes Verfahren, wobei die Saccharoseisomerase-Biomasse aus Protaminobacter rubrum, Klebsiella sp., besonders Stamm LX3 oder Stamm NK33-98-8, Klebsiella pneumoniae, besonders Stamm 342; Enterobacter sp., besonders Stamm SZ62 oder Stamm FMB1, Erwinia tasmaniensis, besonders Stamm Et1/99; Pectobacterium atrosepticum, besonders Stamm SCRI 1043; Pectobacterium carovotum, besonders Subspezies brasiliensis, besonders Stamm PBR 1692, Azotobacter vinelandii, Leucanea leucocephalia, Erwinia rhapontici, Raoultella planticola, Pseudomonas mesoacidophila, Leuconostoc mesenteroides, Pantoea dispersa, Serratia plymuthica, Serratia marcescens oder Agrobacterium radiobacter stammt.

Die vorliegende Erfindung betrifft insbesondere ein erfindungsgemäßes Verfahren, wobei die Verfahrensschritte a) und b) in einem Festbett oder Rührkessel durchgeführt wird.

Die vorliegende Erfindung betrifft insbesondere ein erfindungsgemäßes Verfahren, wobei die erhaltene Isomaltulose-haltige Zusammensetzung katalytisch hydriert wird.

Das erfindungsgemäße Verfahren wird vorzugsweise kontinuierlich durchgeführt, kann aber auch semikontinuierlich oder batch-weise durchgeführt werden.

Die vorliegende Erfindung betrifft insbesondere ein erfindungsgemäßes Verfahren zur Herstellung einer Zuckeralkohol-haltigen Zusammensetzung, wobei ein erfindungsgemäßes Verfahren zur Herstellung einer Isomaltulose-haltigen Zusammensetzung aus einem Saccharose-haltigen Substrat und anschließend eine katalytische Hydrierung der erhaltenen Isomaltulose-haltigen Zusammensetzung durchgeführt und eine Zuckeralkohol-haltige Zusammensetzung erhalten wird.

Die vorliegende Erfindung betrifft auch ein Verfahren zur Herstellung einer Zuckeralkohol-haltigen Zusammensetzung gemäß der vorliegenden Erfindung, wobei die Zuckeralkohol-haltige Zusammensetzung Isomalt oder eine Isomaltvariante ist.

Im Zusammenhang mit der vorliegenden Erfindung wird unter einem "Saccharose-haltigen Substrat" eine Zusammensetzung enthaltend 1 bis 100 Gewichts-%, vorzugsweise 1 bis 99 Gewichts-%, vorzugsweise 10 bis 95 Gewichts-%, insbesondere 20 bis 90 Gewichts-% Saccharose (jeweils bezogen auf Gewicht der Trockensubstanz des Substrats) verstanden.

In einer Ausführungsform kann das Saccharose-haltige Substrat allein aus Saccharose bestehen.

In einer anderen Ausführungsform kann das Saccharose-haltige Substrat zusätzlich zu Saccharose eine, zwei, drei, mehrere oder viele weitere Substanzen enthalten, insbesondere ausgewählt aus der Gruppe bestehend aus Trehalulose, Isomelezitose, Glucose, Fructose, Saccharose, Isomaltose, Trisaccharide und Oligomere.

In einer besonders bevorzugten Ausführungsform weist das Saccharose-haltige Substrat vorzugsweise einen Saccharose-Gehalt vorzugsweise von 2 Gewichts-% bis vorzugsweise zu 85 Gewichts-%, vorzugsweise von 3 Gewichts-% bis vorzugsweise zu 90 Gewichts-%, vorzugsweise von 4 Gewichts-% bis vorzugsweise zu 95 Gewichts-%, vorzugsweise von 5 Gewichts-% bis vorzugsweise zu 96 Gewichts-%, vorzugsweise von 6 Gewichts-% bis vorzugsweise zu 97 Gewichts-%, vorzugsweise von 7 Gewichts-% bis vorzugsweise zu 98 Gewichts-%, vorzugsweise von 8 Gewichts-% bis vorzugsweise zu 99 Gewichts-%, vorzugsweise von 9 Gewichts-% bis vorzugsweise zu 100 Gewichts-%, vorzugsweise von 10 Gewichts-% bis vorzugsweise zu 85 Gewichts-%, vorzugsweise von 20 Gewichts-% bis vorzugsweise zu 90 Gewichts-%, vorzugsweise von 30 Gewichts-% bis vorzugsweise zu 95 Gewichts-%, vorzugsweise von 40 Gewichts-% bis vorzugsweise zu 96 Gewichts-%, vorzugsweise von 50 Gewichts-% bis vorzugsweise zu 97 Gewichts-%, vorzugsweise von 60 Gewichts-% bis vorzugsweise zu 98 Gewichts-%, vorzugsweise von 70 Gewichts-% bis vorzugsweise zu 99 Gewichts-% oder vorzugsweise von 80 Gewichts-% vorzugsweise zu 100 Gewichts-% auf (jeweils bezogen auf Gewicht der Trockensubstanz des Substrats).

In besonders bevorzugter Ausführungsform weist das Saccharose-haltige Substrat einen Gehalt an Saccharose auf vorzugsweise von 5 Gewichts-% bis vorzugsweise zu 75 Gewichts-%, vorzugsweise von 10 Gewichts-% bis vorzugsweise zu 78 Gewichts-%, vorzugsweise von 20 Gewichts-% bis vorzugsweise zu 85 Gewichts-%, vorzugsweise von 30 Gewichts-% bis vorzugsweise zu 88 Gewichts-%, vorzugsweise von 40 Gewichts-% bis vorzugsweise zu 93 Gewichts-%, vorzugsweise von 50 Gewichts-% bis vorzugsweise zu 94 Gewichts-%, vorzugsweise von 60 Gewichts-% bis vorzugsweise zu 95 Gewichts-%, vorzugsweise von 65 Gewichts-% bis vorzugsweise zu 96 Gewichts-%, vorzugsweise von 10 Gewichts-% bis vorzugsweise zu 97 Gewichts-%, vorzugsweise von 20 Gewichts-% bis vorzugsweise zu 98 Gewichts-%, vorzugsweise von 30 Gewichts-% bis vorzugsweise zu 99 Gewichts-%, vorzugsweise von 40 Gewichts-% bis vorzugsweise 100 Gewichts-% (jeweils bezogen auf Gewicht der Trockensubstanz des Substrats).

Besonders bevorzugt ist ein Saccharose-Gehalt von vorzugsweise 60 bis 90 Gewichts-%, vorzugsweise 70 bis 80 Gewichts-%, vorzugsweise 30 bis 60 Gewichts-%, vorzugsweise 40 bis 50 Gewichts-% (jeweils bezogen auf Gewicht der Trockensubstanz des Saccharose-haltigen Substrats) auf.

In besonders bevorzugter Ausführungsform weist das Saccharose-haltige Substrat 90 bis 99 Gewichts-%, vorzugsweise 90 bis 98 Gewichts-%, insbesondere 90 bis 97 Gewichts-% Saccharose (jeweils bezogen auf Gewicht der Trockensubstanz des Substrats) auf.

In besonders bevorzugter Ausführungsform liegt das Saccharose-haltige Substrat in flüssiger Form, vorzugsweise in gelöster oder suspendierter Form, vorzugsweise in einem flüssigen, insbesondere wässrigen, Medium vor. In besonders bevorzugter Ausführungsform ist das wässrige Medium Wasser. In besonders bevorzugter Ausführungsform liegt das Saccharose-haltige Substrat in wässrigem Medium, nämlich in wässriger Lösung oder wässriger Suspension, vor.

In besonders bevorzugter Ausführungsform weist das das Saccharose-haltige Substrat enthaltende wässrige Medium, also z. B. die Lösung oder Suspension, 0,1 bis 80 Gewichts-%, vorzugsweise 1 bis 70 Gewichts-%, vorzugsweise 4 bis 60 Gewichts-%, vorzugsweise 5 bis 50 Gewichts-%, insbesondere 5 bis 40 Gewichts-%, insbesondere von 5 bis 30 Gewichts-%, vorzugsweise von 35 bis 45 Gewichts-%, vorzugsweise von 20 bis 27 %, vorzugsweise 40 bis 75 Gewichts-%, insbesondere 40 bis 60 Gewichts-%, insbesondere 10 bis 60 Gewichts-%, vorzugsweise 20 bis 55 Gewichts-% des Saccharose-haltigen Substrats auf, wobei diese Mengenbereiche mit dem Gewicht des Mediums, also z. B. Wasser, auf 100 % aufaddieren (Gewichts-%, jeweils bezogen auf Gesamtgewicht des Saccharose-Substrat-haltigen Mediums, entspricht dem Trockensubstanzgehalt an Saccharose-haltigem Substrat im Medium).

In besonders bevorzugter Ausführungsform kann die das Saccharose-haltige Substrat enthaltende Lösung oder Suspension Dünnsaft oder Dicksaft aus einer Zucker-verarbeitenden Fabrik sein, vorzugsweise mit einem Trockensubstanzgehalt von 5 bis 70 %, vorzugsweise von 50 bis 70 %, insbesondere von 55 bis 68 %, insbesondere von 5 bis 30 %, vorzugsweise 20 bis 27 % (Gewichts-%, jeweils bezogen auf Gesamtgewicht des Saccharose-Substrat-haltigen Mediums).

Erfindungsgemäß möglich ist auch die Verwendung von Melasse oder anderen unreinen Saccharose-haltigen Zusammensetzungen als Saccharose-haltiges Substrat enthaltendes Medium, insbesondere Lösung oder Suspension.

In einer weiteren bevorzugten Ausführungsform weist das das Saccharose-haltige Substrat aufweisende flüssige Medium, insbesondere die das Saccharose-haltige Substrat aufweisende Lösung oder Suspension, einen Saccharose-Gehalt von 0,1 bis 80 Gewichts-%, 5 bis 30 Gewichts-%, 20 bis 30 Gewichts-%, 20 bis 60 Gewichts-%, 30 bis 60 Gewichts-%, 35 bis 45 Gewichts-%, 40 bis 75 Gewichts-%, 40 bis 60 Gewichts-%, 10 bis 60 Gewichts-% oder vorzugsweise 20 bis 55 Gewichts-% (jeweils bezogen auf Gesamtgewicht des Saccharose-haltigen flüssigen Mediums) auf.

Im Zusammenhang mit der vorliegenden Erfindung wird unter einer "Isomaltulose-haltigen Zusammensetzung" das Isomerisierungsprodukt der Aktivität einer Saccharoseisomerase auf ein Saccharose-haltiges Substrat verstanden. Eine Isomaltulose-haltige Zusammensetzung der vorliegenden Erfindung weist ein Gemisch aus Isomaltulose und Trehalulose auf, insbesondere besteht aus diesen. Bevorzugt ist in einer Ausführungsform mehr Isomaltulose als Trehalulose und in einer anderen Ausführungsform mehr Trehalulose als Isomaltulose vorhanden. Weitere Bestandteile einer Isomaltulose-haltigen Zusammensetzung können Isomelezitose, Fructose, Glucose, Isomaltose, Saccharose, Trisaccharide, Oligomere oder Mischungen von zwei oder mehreren dieser Zucker sein.

Besonders bevorzugt beträgt der Anteil der Isomaltulose in der Isomaltulose-haltigen Zusammensetzung zumindest 80 Gewichts-%, vorzugsweise mindestens 81 Gewichts-%, mindestens 82 Gewichts-%, mindestens 83 Gewichts-%, mindestens 84 Gewichts-%, mindestens 85 Gewichts-%, bevorzugt mindestens 86 Gewichts-% und insbesondere mindestens 87 Gewichts-%, mindestens 88 Gewichts-%, mindestens 89 Gewichts-%, mindestens 90 Gewichts-%, mindestens 91 Gewichts-%, mindestens 92 Gewichts-% oder 93 Gewichts-% (jeweils bezogen auf Trockensubstanz der Isomaltulose-haltigen Zusammensetzung). Die auf 100 % aufaddierenden Restbestandteile einer solchen Isomaltulose-haltigen Zusammensetzung werden durch Trehalulose sowie optional Isomelezitose, Fructose, Glucose, Saccharose, Isomaltose, Trisaccharide, Oligomere oder zwei oder mehreren davon gebildet.

In besonders bevorzugter Ausführungsform weist die Isomaltulose-haltige Zusammensetzung 60 bis 98 Gewichts-%, insbesondere 70 bis 95 Gewichts-%, vorzugsweise 75 bis 88 Gewichts-%, insbesondere 75 bis 84 Gewichts-% Isomaltulose (jeweils bezogen auf Trockensubstanz der Isomaltulose-haltigen Zusammensetzung) auf.

In einer weiteren bevorzugten Ausführungsform kann die Isomaltulose-haltige Zusammensetzung von 1 bis 20 Gewichts-%, 5 bis 20 Gewichts-%, 10 bis 30 Gewichts-% oder 20 bis zu 99 Gewichts-%, vorzugsweise 20 bis 40 Gewichts-%, vorzugsweise 20 bis 30 Gewichts-%, vorzugsweise von 30 bis vorzugsweise zu 95 Gewichts-%, vorzugsweise von 45 zu 96 Gewichts-%, vorzugsweise von 46 zu 97 Gewichts-%, vorzugsweise von 47 zu 98 Gewichts-%, vorzugsweise von 48 zu 99 Gewichts-%, vorzugsweise von 49 zu 99 Gewichts-%, vorzugsweise von 40 bis zu 98 Gewichts-%, vorzugsweise von 50 bis 97 Gewichts-%, von 60 bis 96 Gewichts-% oder von vorzugsweise 70 bis zu 97 Gewichts-%, vorzugsweise von 80 bis zu 98 Gewichts-%, vorzugsweise von 75 bis zu 98 Gewichts-%, insbesondere 50 bis 99 Gewichts-%, insbesondere 60 bis 98 Gewichts-%, insbesondere von 85 bis zu 99 Gewichts-% und vorzugsweise 95 Gewichts-% bis 99 Gewichts-%, vorzugsweise von 96 bis 99 Gewichts-%, vorzugsweise von 97 bis 99 Gewichts-%, vorzugsweise von 98 bis 99 Gewichts-% Trehalulose (jeweils bezogen auf Trockensubstanz der Isomaltulose-haltigen Zusammensetzung) enthalten. Die auf 100 Gewichts-% der Gesamtzusammensetzung aufaddierenden Restbestandteile werden durch Isomaltulose sowie optional Glucose, Fructose, Isomelezitose, Isomaltose, Saccharose, Trisaccharide, Oligomere oder zwei oder mehrere davon gebildet.

In besonders bevorzugter Ausführungsform weist die Isomaltulose-haltige Zusammensetzung 60 bis 90 Gewichts-% Isomaltulose, 5 bis 40 Gewichts-% Trehalulose und 0 bis 5 Gewichts-% mindestens einer weiteren Substanz ausgewählt aus der Gruppe bestehend aus Isomelezitose, Saccharose, Fructose, Glucose, Isomaltose, Trisaccharide und Oligomere auf.

In einer weiteren bevorzugten Ausführungsform weist die vorliegende Isomaltulose-haltige Zusammensetzung 60 bis 90 Gewichts-% Trehalulose, 5 bis 40 Gewichts-% Isomaltulose und 0 bis 5 Gewichts-% mindestens einer weiteren Substanz ausgewählt aus der Gruppe bestehend aus Isomelezitose, Fructose, Glucose, Saccharose, Isomaltose, Trisaccharide und Oligomere auf.

In einer weiteren bevorzugten Ausführungsform liegt die Isomaltulose-haltige Zusammensetzung in flüssiger Form, vorzugsweise in gelöster oder suspendierter Form, vorzugsweise in einem flüssigen, insbesondere wässrigen, Medium vor. In besonders bevorzugter Ausführungsform ist das wässrige Medium Wasser. In besonders bevorzugter Ausführungsform liegt die Isomaltulose-haltige Zusammensetzung in wässrigem Medium, nämlich in wässriger Lösung oder wässriger Suspension, vor.

In besonders bevorzugter Ausführungsform weist das die Isomaltulose-haltige Zusammensetzung enthaltende wässrige Medium, also z. B. die Lösung oder Suspension, 0,1 bis 80 Gewichts-%, vorzugsweise 1 bis 70 Gewichts-%, vorzugsweise 4 bis 60 Gewichts-%, vorzugsweise 5 bis 50 Gewichts-%, insbesondere 5 bis 40 Gewichts-%, insbesondere von 5 bis 30 Gewichts-%, vorzugsweise von 35 bis 45 Gewichts-%, vorzugsweise von 20 bis 27 %, vorzugsweise 40 bis 75 Gewichts-%, insbesondere 40 bis 60 Gewichts-%, insbesondere 10 bis 60 Gewichts-%, vorzugsweise 20 bis 55 Gewichts-% der Isomaltulose-haltigen Zusammensetzung auf, wobei diese Mengenbereiche mit dem Gewicht des Mediums, also z. B. Wasser, auf 100 % aufaddieren (Gewichts-%, jeweils bezogen auf Gesamtgewicht des die Isomaltulose-haltige Zusammensetzung enthaltenden Mediums, entspricht dem Trockensubstanzgehalt an Isomaltulose-haltiger Zusammensetzung im Medium).

Im Zusammenhang mit der vorliegenden Erfindung ist die "Saccharoseisomerase-Biomasse" eine Biomasse, insbesondere eine Zelle, ein Zellextrakt, ein Enzym oder ein Enzymgemisch, wobei die Biomasse in der Lage ist, unter geeigneten Bedingungen ein Saccharose-haltiges Substrat in eine Isomaltulose-haltige Zusammensetzung zu konvertieren, das heißt eine Saccharoseisomerase-Aktivität aufweist. Vorzugsweise weist die Saccharoseisomerase eine $\alpha$-Glucosyl-Transferase oder Saccharose-6-glucosyl-mutase (EC 5.4.99.11)-Aktivität auf.

In vorteilhafter Weise können die als Saccharoseisomerase-Biomasse einsetzbaren Zellen lebend oder tot sein. In besonders bevorzugter Ausführungsform können die als Saccharoseisomerase-Biomasse eingesetzten Zellen vollständige oder zerstörte, insbesondere aufgeschlossene Zellen, Zellfragmente, Zelllysate oder Zellextrakte sein. Die erfindungsgemäß eingesetzte Saccharoseisomerase-Biomasse kann in bevorzugter Ausführungsform aufgereinigt, insbesondere isoliert sein, beispielsweise durch herkömmliche Aufreinigungsverfahren wie Lösemittelextraktion, French Press, Lyophilisation und/oder enzymatische Behandlung. Die eingesetzte Saccharoseisomerase-Biomasse kann natürlichen Ursprungs sein oder sie kann in einer Ausführungsform gentechnisch verändert sein.

Der erfindungsgemäß vorgesehene Verfahrensschritt a) des Inkontaktbringens des Saccharose-haltigen Substrates mit einer Träger-immobilisierten Saccharoseisomerase-Biomasse wird erfindungsgemäß bevorzugt so durchgeführt, dass eine Umsetzung des Saccharose-haltigen Substrats zu einer Isomaltulose-haltigen Zusammensetzung stattfinden kann, vorzugsweise in einer Umsatz-optimierten Weise.

Vorzugsweise wird das Inkontaktbringen bei einer Temperatur von 10 bis 40°C, vorzugsweise 15 bis 40°C, vorzugsweise 10 bis 37°C, vorzugsweise 25 bis 40°C, vorzugsweise 25 bis 30°C, vorzugsweise 30 bis 40°C, vorzugsweise 10 bis 25°C, vorzugsweise 15 bis 30°C, vorzugsweise 18 bis 26°C, vorzugsweise 10 bis 20°C, insbesondere 10 bis 17°C durchgeführt.

In einer weiteren bevorzugten Ausführungsform ist vorgesehen, dass der Verfahrensschritt a) bei einem pH-Wert von 5,0 bis 9,0, vorzugsweise 5,0 bis 7,0, vorzugsweise 6,0 bis 7,0 durchgeführt wird.

Im Zusammenhang mit der vorliegenden Erfindung wird unter einer Träger-immobilisierten Saccharoseisomerase-Biomasse eine an oder in einem Träger immobilisierte Saccharoseisomerase-Biomasse verstanden. Diese wird im Folgenden kurz auch als Immobilisat bezeichnet. Die Immobilisierung kann in herkömmlicher Weise, zum Beispiel durch Adsorption, Bindung, insbesondere kovalente Bindung, Quervernetzung, Verkapselung oder Einschlussimmobilisierung erfolgt sein.

In besonders bevorzugter Weise sieht die vorliegende Erfindung vor, die partikuläre Träger-immobilisierte Saccharoseisomerase-Biomasse herzustellen, indem in einem ersten Verfahrensschritt x1) Biomasse bereitgestellt wird, beispielsweise durch Gewinnung in einem Fermenter, und diese Biomasse in einem zweiten Verfahrensschritt x2) mit einer Alginat-Lösung vermischt und in einem dritten Verfahrensschritt x3) die Biomasse-Alginat-Mischung in eine Calciumsalz-Lösung, insbesondere eine Calciumchlorid- oder Calciumacetat-Lösung, eingebracht, insbesondere eingetropft wird. Die sich bildenden Partikel gelieren und werden in einem vierten Verfahrensschritt x4) getrocknet, beispielsweise in einem Wirbelschichttrockner.

In besonders bevorzugter Ausführungsform kann vorgesehen sein, die in dem ersten Verfahrensschritt x1) bereitgestellte Biomasse vor Vermischen mit der Alginat-Lösung aufzukonzentrieren, das heißt einzudicken.

In besonders bevorzugter Ausführungsform ist vorgesehen, in Verfahrensschritt x3) die Biomasse-Alginat-Mischung in eine Calciumsalz-Lösung durch Eintropfen unter Druck insbesondere mittels Druckluft, das heißt in einem Abblasverfahren, einzubringen. In einer weiteren bevorzugten Ausführungsform kann in Verfahrensschritt x3) die Biomasse-Alginat-Mischung in die Calciumsalz-Lösung mittels eines elektrostatischen Verfahrens, insbesondere unter Anlegen einer Spannung an die Calciumsalz-Lösung und die Biomasse-Alginat-Mischung, eingetropft werden. In einer weiteren Ausführungsform kann Verfahrensschritt x3) durchgeführt werden, indem die Biomasse-Alginat-Mischung, beispielsweise mittels eines Stempels, in Vibration versetzt wird und so ein gezieltes Eintropfen dieser Mischung in die Calciumsalz-Lösung erfolgt. Gegebenenfalls kann ein erhöhter Druck, beispielsweise ein erhöhter Luftdruck, eingesetzt werden.

In einer weiteren Ausführungsform kann in Verfahrensschritt x3) die Biomasse-Alginat-Mischung unter Nutzung eines seitlichen Abblasdrucks in die Calciumsalz-Lösung eingetropft werden. In einer weiteren bevorzugten Ausführungsform kann in Verfahrensschritt x3) die Biomasse-Alginat-Mischung auf eine rotierende Scheibe aufgebracht und so in die Calciumsalz-Lösung eingetropft werden. In einer weiteren bevorzugten Ausführungsform kann in Verfahrensschritt x3) die Biomasse-Alginat-Mischung durch rotierende Düsen in die Calciumsalz-Lösung eingetropft werden.

In einer weiteren bevorzugten Ausführungsform kann in Verfahrensschritt x3) die Biomasse-Alginat-Mischung über einen Strahlschneider/Jet-Cutter® in die Calciumsalz-Lösung eingetropft werden. In einer weiteren bevorzugten Ausführungsform kann die Biomasse-Alginat-Mischung im Verfahrensschritt x3) durch Mehrfach-Düsen-Systeme in Kombination mit Bandtrocknern und einer Lufttrocknung, optional unter Einsatz von Schneidwerkzeugen, in die Calciumsalz-Lösung eingetropft werden.

Die vorgenannten Verfahrensschritte, insbesondere die vorstehend beschriebenen Immobilisierungstechniken gemäß Verfahrensschritt x3), führen in vorteilhafter Weise zur Bereitstellung der erfindungsgemäß bevorzugten Partikeldurchmesser dp.

Im Zusammenhang mit der vorliegenden Erfindung wird unter einer partikulären Träger-immobilisierten Saccharoseisomerase-Biomasse eine solche Träger-immobilisierte Saccharoseisomerase-Biomasse verstanden, die in Partikelform vorliegt, das heißt die in Form von Teilchen vorliegen, insbesondere in Form von festen Teilchen.

Die partikuläre Träger-immobilisierte Saccharoseisomerase-Biomasse weist in trockener Form Partikeldurchmesser dp von 100 bis 1300 $\mu$m, vorzugsweise 200 bis 1300 $\mu$m, vorzugsweise 150 bis 1200 $\mu$m, vorzugsweise 150 bis 1000 $\mu$m, vorzugsweise 100 bis 900 $\mu$m, vorzugsweise 150 bis 950 $\mu$m, vorzugsweise 150 bis 900 $\mu$m, vorzugsweise 150 bis 850 $\mu$m, insbesondere 100 bis 800 $\mu$m, vorzugsweise 200 bis 800 $\mu$m, insbesondere 200 bis 950 $\mu$m, insbesondere 250 bis 500 $\mu$m, insbesondere 300 bis 700 $\mu$m, vorzugsweise 400 bis 600 $\mu$m, insbesondere 450 bis 550 $\mu$m, insbesondere 480 bis 540 $\mu$m, vorzugsweise 500 $\mu$m auf (jeweils bezogen auf trockene Träger-immobilisierte Saccharoseisomerase-Biomasse).

Offenbart wird insbesondere ein Verfahren, wobei die partikuläre Träger-immobilisierte Saccharoseisomerase-Biomasse einen Partikeldurchmesser dp von 400 bis 600 $\mu$m, insbesondere 100 bis 400 $\mu$m aufweist.

Des Weiteren weist die partikuläre Träger-immobilisierte Saccharoseisomerase-Biomasse einen Partikeldurchmesser dp von 250 bis 500 $\mu$m auf.

Des Weiteren weist die partikuläre Träger-immobilisierte Saccharoseisomerase-Biomasse in trockener Form einen volumetrischen Mittelwert D[4,3] in einem Bereich von 380 bis 570 $\mu$m auf, insbesondere einen volumetrischen Mittelwert D[4,3] in einem Bereich von 380 bis 400 $\mu$m, 450 bis 490 $\mu$m oder 530 bis 580 $\mu$m.

Des Weiteren weist die partikuläre Träger-immobilisierte Saccharoseisomerase-Biomasse in trockener Form einen D[3,2]-Wert in einem Bereich von 360 bis 540 $\mu$m, insbesondere 360 bis 380, 430 bis 450 oder 510 bis 530 $\mu$m auf.

Des Weiteren weist die partikuläre Träger-immobilisierte Saccharoseisomerase-Biomasse in trockener Form eine spezifische Oberfläche in einem Bereich von 0,0100 bis 0,0180, insbesondere in einem Bereich von 0,0100 bis 0,0118 oder 0,0130 bis 0,0140 oder 0,0150 bis 0,0170 m$^2$/g auf.

Des Weiteren weist die partikuläre Träger-immobilisierte Saccharoseisomerase-Biomasse in trockener Form eine Gleichförmigkeit in einem Bereich von 0,190 bis 0,210 auf.

Des Weiteren weist die partikuläre Träger-immobilisierte Saccharoseisomerase-Biomasse einen d(0,1)-Wert in einem Bereich von 270 bis 400 $\mu$m, insbesondere in einem Bereich von 270 bis 290 $\mu$m, 320 bis 340 $\mu$m oder 380 bis 400 $\mu$m auf.

Die partikuläre Träger-immobilisierte Saccharoseisomerase-Biomasse weist in trockener Form einen d(0,5)-Wert in einem Bereich von 370 bis 550 $\mu$m, insbesondere 370 bis 390 $\mu$m, 450 bis 470 $\mu$m oder 530 bis 550 $\mu$m auf.

In einer weiteren bevorzugten Ausführungsform weist die partikuläre Träger-immobilisierte Saccharoseisomerase-Biomasse in trockener Form eine d(0,9)-Wert in einem Bereich von 510 bis 530 $\mu$m auf.

Sofern in der vorliegenden Lehre nicht anders angegeben, beziehen sich alle für die Charakterisierung der partikulären Träger-immobilisierten Saccharoseisomerase-Biomasse angegebenen Parameter wie die spezifische Oberfläche, die Partikeldurchmesser, die volumetrischen Mittelwerte oder die d(0,1)-, d(0,5)-, d(0,9)-Werte auf in trockener Form gemessene Partikel.

Die Beschreibung offenbart auch eine partikuläre Träger-immobilisierte Saccharoseisomerase-Biomasse in trockener Form mit einem Partikeldurchmesser dp von 100 bis 500, insbesondere 100 bis 450, insbesondere 100 bis 400, insbesondere 100 bis 300, insbesondere 200 bis 300, bevorzugt 250 bis 300 $\mu$m.

Im Zusammenhang mit der vorliegenden Erfindung wird unter dem Begriff "d(0,5)-Wert" oder dem Begriff "Partikeldurchmesser d(0,5)" der Medianwert der Partikelgrößenverteilung bezogen auf die Massen- oder Volumenverteilung verstanden, wobei d(0,5) bedeutet, dass 50 % der Partikelmasse kleiner ist als der jeweils angegebene Wert oder.

Analog wird unter dem Begriff d(0,1) beziehungsweise d(0,9) verstanden, dass 10 beziehungsweise 90 % der Partikelmasse kleiner ist als der jeweils angegebene Wert.

Die Partikelgrößenverteilung ist eine volumetrische Partikelgrößenverteilung.

In besonders bevorzugter Ausführungsform erfolgt die Bestimmung der Partikelgrößenverteilung der hergestellten partikulären Träger-immobilisierten Saccharoseisomerase-Biomasse mittels Laserbeugung, insbesondere gemäß der Verfahrensweise aus Beispiel 1 der vorliegenden Lehre.

In besonders bevorzugter Ausführungsform ist die partikuläre Träger-immobilisierten Saccharoseisomerase-Biomasse kugelförmig.

Die Träger-immobilisierte Saccharoseisomerase-Biomasse weist in bevorzugter Ausführungsform einen Gleichmäßigkeitskoeffizient < 1,2 auf.

Der Gleichmäßigkeitskoeffizient wird gemäß folgender Formel berechnet.

$$Gleichmäßigkeitskoeffizient = \frac{\sum X_i |d(x,0.5) - d_i|}{d(x,0.5) \sum X_i}$$

In besonders bevorzugter Ausführungsform weist die Träger-immobilisierte Saccharoseisomerase-Biomasse eine spezifische Aktivität von mindestens 400 units/g TS, vorzugsweise mindestens 450 units/g TS, vorzugsweise mindestens 500 units/g TS, insbesondere mindestens 600 units/g TS, insbesondere mindestens 700 units/g TS, vorzugsweise mindestens 800 units/g TS auf.

Die Beschreibung offenbart auch ein Verfahren gemäß der vorstehenden Verfahrensschritte a) und b) zum Erhalt einer Isomaltulose-haltigen Zusammensetzung, wobei die partikuläre Träger-immobilisierte Saccharoseisomerase-Biomasse einen Partikeldurchmesser dp von 100 bis 1300 $\mu$m aufweist, insbesondere von 150 bis 1300 $\mu$m, vorzugsweise von 100 bis 900 $\mu$m (jeweils gemessen in trockener Form). Ein Verfahren der vorliegenden Erfindung ist auch eine besondere vorteilhafte Ausgestaltung eines solchen Verfahrens, wobei die verschiedenen besonderen Ausgestaltungen des erfindungsgemäßen Verfahrens auch miteinander kombiniert werden können, sofern sich diese Kombinationen nicht technisch ausschließen.

Weitere vorteilhafte Ausgestaltungen der Erfindung ergeben sich aus den Unteransprüchen.

Die Erfindung wird anhand der folgenden Beispiele und der dazugehörigen Figuren näher erläutert.

Die Figuren zeigen:

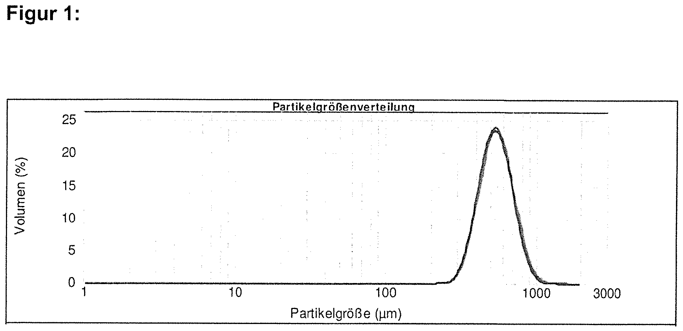

Figur 1 zeigt graphisch die Größenverteilung einer ersten erfindungsgemäßen Partikelpräparation (Grobkorn),

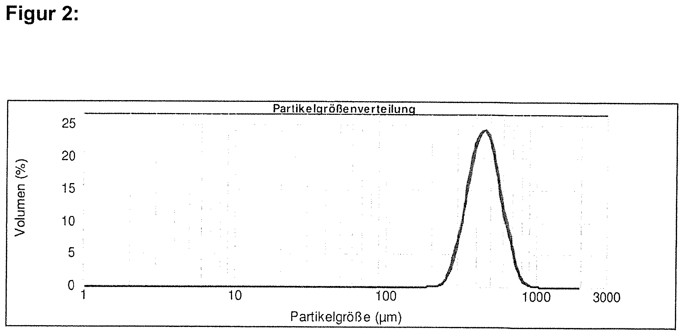

Figur 2 die Größenverteilung einer zweiten erfindungsgemäßen Partikelpräparation (Normalkorn) und

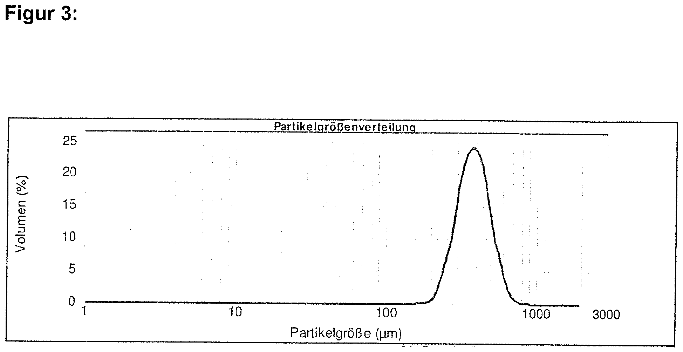

Figur 3 die Größenverteilung einer dritten erfindungsgemäßen Partikelpräparation (Feinkorn).

Beispiel 1

Bestimmung der Partikelgrößenverteilung von immobilisierten Biokatalysatoren mittels Laserbeugung mit dem Mastersizer 2000, Fa. MALVERN Trockenmessung

Durchführung

Mit Hilfe der Laserbeugungsmessung wird die Partikelgrößenverteilung des erfindungsgemäß hergestellten Biokatalysators (Immobilisates) ermittelt. Zur Beschreibung der Größenverteilung werden die Parameter $d_{0,1}$, $d_{0,5}$, $d_{0,9}$ und die Gleichförmigkeit ausgewiesen.

Kurzbeschreibung

Das Messprinzip beruht auf der Streulicht/Laserbeugungsspektroskopie nach ISO 13320. Vereinzelte Partikel werden in geringer Konzentration in einen Laserstrahl gebracht. Das Einbringen der Partikel erfolgt durch Einsaugen der trockenen Biokatalysatorprobe in die Messzelle ("Trockenmessung"). Abhängig vom Durchmesser der Partikel kommt es zu einer Beugung des Laserlichtes, die als Streustrahlung von Detektoren aufgefangen wird. Das Messergebnis liegt zunächst in der Form der an den Detektoren gemessenen Lichtintensitäten vor und muss in eine Partikelgrößenverteilung umgerechnet werden. Dies geschieht mittels der üblichen Auswertungssoftware für die hier gegebenen Partikel > 1$\mu$m durch eine Näherung von Joseph von Fraunhofer (1814).

Geräte/Hilfsmittel

- Analysensieb mit einem Durchmeser von 200 mm nach DIN ISO 3310-1 der Maschenweite 1,25 mm

- Auffangboden und Deckel für das Analysensieb

- Malvern Mastersizer 2000 mit Trockendispergiereinheit Scirocco 2000 (A) einschließlich zugehöriger Steuerungs- und Auswertungssoftware, Fa. Malvern Instruments Ltd.

Probenvorbereitung

- Alle Wägungen werden auf $\pm$0,01 g (Ablesegenauigkeit) durchgeführt.

- Leergewichte (Tara) des Siebes und des Auffangbodens ermitteln

- Auffangboden und Sieb zusammensetzen

- Gewicht der gesamten Biokatalysator- Probe (in der Regel in einem 250-mL-Fläschchen) der jeweiligen Biokataysator-Charge ermitteln.

- Probe quantitativ auf das Sieb überführen, Deckel aufsetzen und von Hand durchsieben

- Nach dem Durchsieben sowohl das Sieb mit dem Rückstand als auch den Auffangboden mit dem Durchgang auswiegen (Brutto)

- Auswertung/Berechnung: Aus dem Siebrückstand wird der prozentuale Grobanteil der Biokatalysator-Probe ermittelt

- Der Siebrückstand wird verworfen und der Durchgang im Auffangboden in die Original-Probenflasche zurückgegeben

Messung mit dem Malvern Mastersizer 2000

Dosierung/Zuführung der Probe

Die Dosierung der Proben wird über eine Schüttelrinne mit verstellbarer Spaltbreite vorgenommen. Als Siebeinsatz wird ein grobes Sieb mit mehreren Kugeln eingesetzt.

Die Dosierung (Spaltbreite) muss je nach Produkt so eingestellt werden, dass die Messkonzentration (grüner Bereich) erreicht wird.

Dispergiermedium

Als Dispergiermedium wird Luft mit einem bestimmten Druck eingesetzt.

Die Durchführung der Messungen erfolgt mit dem Mastersizer 2000, Dispergiereinheit Scirocco 2000 (A).

Beispiel 2:

A. Herstellung des Biokatalysators

Von einer Abimpfung des Stammes Protaminobacter rubrum (CBS 574.77) werden Zellen mit 10 ml eines sterilen Nährsubstrates, bestehend aus 8 kg Dicksaft aus einer Zuckerfabrik (Trockensubstanzgehalt = 65 %), 2 kg Maisquellwasser, 0,1 kg $(NH_4)_2$ $HPO_4$ und 89,9 kg dest. Wasser, bei Bedarf auf pH 7,2 eingestellt, abgeschwemmt. Diese Suspension dient als Impfgut für die Schüttelmaschinen-Vorkultur in 1-Liter-Kolben mit 200 ml Nährlösung obiger Zusammensetzung.

Nach einer 30-stündigen Bebrütungszeit bei 29 °C werden mit je 10 Kolben (Gesamtinhalt 2 Liter) 18 Liter Nährlösung obiger Zusammensetzung in einem 30-Liter-Kleinfermenter beimpft und bei 29 °C mit 20 Liter Luft pro Minute und einer Rührgeschwindigkeit von 350 UpM fermentiert.

Nach Erreichen von Keimzahlen über 5 x $10^9$ Keimen/ml wird die Fermentation abgestellt; die Zellen werden durch Zentrifugation aus der Fermenterlösung abgeerntet und in einer 2%-igen Natriumalginatlösung suspendiert.

Durch Verwendung einer Rotationsimmobilisierung, von Strahlschneideverfahren oder pulsierenden Tropfverfahren, insbesondere unter Einsatz von elektrostatischen Verfahren, Abblas- oder Vibrationsverfahren, insbesondere unter Einsatz eines seitlichen Abblasdruckes, rotierender Scheiben oder rotierender Düsen, wird die Suspension in eine 2%-ige Calciumchloridlösung eingetropft und die erfindungsgemäß bevorzugten Partikeldurchmesser und Partikelgrößenverteilungen, insbesondere mit einem Partikeldurchmesser dp von 250-500 $\mu$m (bezogen auf trockene Partikel) bereitgestellt. Eingesetzt werden auch Mehrfachdüsensysteme unter Einsatz eines Bandtrockners.

Tabelle 1 stellt für drei beispielhaft erhaltene Biokatalysator-Präparationen die erhaltenen charakterisierenden Parameter dar, und zwar für eine erste Präparation, bezeichnet als Grobkorn, eine zweite Präparation, bezeichnet als Normalkorn und eine dritte Präparation, bezeichnet als Feinkorn.

Tabelle 1

|  | d(0,1) (µm) | d(0,5) (µm) | d(0,9) (µm) | Vol. Mittelwert D[4,3] (µm) | D[3,2] (µm) | Spez. Oberfläche (m²/g) | Gleichförmigkeit |
|---|---|---|---|---|---|---|---|
| Grobkorn | 392.690 | 539.575 | 749.682 | 559.012 | 524.745 | 0.0114 | 0.207 |
| Normalkorn | 335.826 | 457.546 | 626.839 | 470.798 | 444.496 | 0.0135 | 0.198 |
| Feinkorn | 280.522 | 381.883 | 523.390 | 393.435 | 371.278 | 0.0162 | 0.202 |

Die entstandenen Immobilisatkugeln werden mit Wasser gewaschen. Dieser Biokatalysator ist bei + 4 °C mehrere Wochen lagerfähig.

B. Herstellung der Isomaltulose-haltigen Zusammensetzung

Die wie unter A erhaltenen immobilisierten Zellen werden in einen temperierbaren Säulenreaktor gefüllt, auf 25 bis 30 °C temperiert und mit einer Saccharoselösung mit 35 bis 45 % TS-Gehalt kontinuierlich durchströmt. Die Fliessgeschwindigkeit wird dabei so eingestellt, dass mindestens 97 % der eingesetzten Saccharose umgelagert werden.

Tabelle 2

| Eingesetzter Biokatalysator | Feedstrom [mL/h] | Umsatzrate gTS Saccharose/ gTS Immobilisat/h | rel. Aktivität % |
|---|---|---|---|
| Mittelwert Grobkorn | 120 | 2,3 | 95 |
| Mittelwert Normalkorn | 126 | 2,4 | 100 |
| Mittelwert Feinkorn | 149 | 2,8 | 118 |
| Produktion (800 kg TS) | 4500 l/h | 2,25 | 94 |

Tabelle 2 zeigt die Katalysatoraktivität von Immobilisaten unterschiedlicher Partikeldurchmesser gemäß Tabelle 1 in einem 100 ml Festbett.

Eine HPLC-Analyse der aus dem Säulenreaktor austretenden Isomaltulose-haltigen Zusammensetzung ergab folgende Zusammensetzung:

Tabelle 3

| Fructose | Glucose | Saccharose | Isomaltulose | Trehalulose | Isomaltose | DP-3 | Isomelizitose | Restsaccharide |
|---|---|---|---|---|---|---|---|---|
| 2,7 | 2,0 | 0,9 | 85,1 | 8,0 | 0,8 | 0,3 | 0,3 | <0,1 |
| 2,7 | 2,0 | 0,9 | 85,2 | 8,0 | 0,7 | 0,2 | 0,2 | <0,1 |
| 2,7 | 2,0 | 0,9 | 85,3 | 7,8 | 0,8 | 0,2 | 0,3 | <0,1 |

Produktspektrum gemäß Tabelle 2 (Grobkorn, Normalkorn, Feinkorn) (Angaben in g/100 g Trockensubstanz).

D. Hydrierung der Isomaltulose-haltigen Zusammensetzung

Die jeweils von der Rest-Saccharose befreiten Ansätze der Isomaltulose-haltigen Zusammensetzung wurden an Raney-Nickel bei 80 °C mit Wasserstoffgas unter Druck von etwa 10 MPa kontinuierlich hydriert. Nach Nickel-Abtrennung und Reinigung durch Ionenaustausch hatten die Ansätze der unter neutralen Bedingungen hydrierten Isomaltulose-haltigen Zusammensetzung etwa die folgende Zusammensetzung:

Tabelle 4

| Mannit | 1,5 % a. TS |
|---|---|
| Sorbit | 4,0 % a. TS |
| 1,6-GPS | 44,4 % a. TS |
| 1,1-GPS | 3,8 % a. TS |
| 1,1-GPM | 45,3 % a. TS |
| hydrierte und nicht-hydrierte Oligomere | 1,0 % a. TS |

Beispiel 3:

Zur Herstellung dieses Biokatalysators wurden von einer Abimpfung des Stammes Pseudomonas mesoacidophila MX-45 (FERM 11808) Zellen mit 10 ml eines sterilen Nährsubstrates aus 8 kg Dicksaft von einer Zuckerfabrik (Trockensubstanzgehalt = 65 %), 2 kg Maisquellwasser, 0,1 kg $(NH_4)_2\,HPO_4$ und 89,9 kg destilliertem Wasser auf einen pH-Wert von 7,2 eingestellt, abgeschwemmt. Diese Suspension dient als Impfgut für eine Schüttelmaschinen-Vorkultur in einem 1-Liter-Kolben mit 200 ml der Nährlösung.

Nach einer 30-stündigen Bebrütung bei 29 °C wurden mit je 10 Kolben (Gesamtinhalt 2 Liter) 18 Liter Nährlösung obiger Zusammensetzung in einem 30-Liter-Kleinfermenter beimpft und bei 29 °C mit 20 Liter Luft pro Minute und einer Rührgeschwindigkeit von 350 Upm fermentiert.

Nach Erreichen von Keimzahlen über $5 \times 10^9$ Keimen/ml wurde die Fermentation abgestellt, die Zellen durch Zentrifugation aus der Fermenterlösung abgeerntet und in einer 2%-igen Natriumalginatlösung suspendiert.

Durch Verwendung einer Rotationsimmobilisierung, von Strahlschneideverfahren oder pulsierenden Tropfverfahren, insbesondere unter Einsatz von elektrostatischen Verfahren, Abblas- oder Vibrationsverfahren, insbesondere unter Einsatz eines seitlichen Abblasdruckes, rotierender Scheiben oder rotierender Düsen, wird die Suspension in eine 2%-ige Calciumchloridlösung eingetropft und die erfindungsgemäß bevorzugten Partikeldurchmesser und Partikelgrößenverteilungen, insbesondere mit einem Partikeldurchmesser dp gemäß Tabelle 1 (bezogen auf trockene Partikel) bereitgestellt. Eingesetzt werden auch Mehrfachdüsensysteme unter Einsatz eines Bandtrockners.

Die entstandenen Immobilisatkugeln wurden mit Wasser gewaschen. Dieser Biokatalysator ist bei + 4 °C mehrere Wochen lagerfähig.

Zur Herstellung von Isomaltulose-haltiger Zusammensetzung wurden die derart erhaltenen immobilisierten Zellen von Pseudomonas mesoacidophila MX-45 (FERM 11808) in einen temperierbaren Säulenreaktor gefüllt, auf etwa 25 bis 30 °C temperiert und mit einer Saccharoselösung mit etwa 35 bis 45 % TS-Gehalt kontinuierlich durchströmt. Die Fliessgeschwindigkeit wurde dabei so eingestellt, dass mindestens 97 % der eingesetzten Saccharose umgelagert wurden.

Eine HPLC-Analyse der aus dem Säulenreaktor austretenden Isomaltulose-haltigen Zusammensetzung ergab folgende Zusammensetzung:

Tabelle 5

| Fructose | 0,2 % a. TS |
|---|---|
| Glucose | 0,2 % a. TS |
| Saccharose | 1,0 % a. TS |
| Isomaltulose | 12,5 % a. TS |
| Isomaltose | 0,2 % a. TS |
| Trehalulose | 85,7 % a. TS |

(fortgesetzt)

| Oligomere (DP > 3) | 0,2 % a. TS |
|---|---|

Die derart hergestellte Isomaltulose-haltige Zusammensetzung wurde von Rest-Saccharose befreit und an Raney-Nickel bei etwa 80 °C mit Wasserstoffgas unter Druck bei 8 bis 12 MPa kontinuierlich hydriert.

Nach Nickel-Abtrennung und Reinigung durch Ionenaustausch hatte die unter neutralen Bedingungen hydrierte Isomaltulose-haltige Zusammensetzung die folgende Zusammensetzung:

Tabelle 6

| Mannit | 0,4 % a. TS |
|---|---|
| Sorbit | 1,0 % a. TS |
| 1,1-GPM | 57,7 % a. TS |
| 1,1-GPS | 34,4 % a. TS |
| 1,6-GPS | 6,4 % a. TS |
| hydrierte und nicht-hydrierte Oligomere | 0,2 % a. TS |

Um die hydrierten und nicht-hydrierten Oligomeren sowie Sorbit durch chromatographische Trennung aus dem Produkt zu entfernen, wurde die chromatographische Trennung nach der Hydrierung mit einer chromatographischen Trennsäule mit einem mit Natrium- bzw. Kaliumionen beladenen starksauren Kationenaustauscherharz durchgeführt.

**Patentansprüche**

1. Verfahren zur Herstellung einer Isomaltulose-haltigen Zusammensetzung aus einem Saccharose-haltigen Substrat, umfassend die folgenden Verfahrensschritte:

   a) Inkontaktbringen des Saccharose-haltigen Substrates mit einer partikulären Träger-immobilisierten Saccharoseisomerase-Biomasse und
   b) Erhalten einer Isomaltulose-haltigen Zusammensetzung,

   **dadurch gekennzeichnet, dass** die Träger-immobilisierte Saccharoseisomerase-Biomasse einen Medianwert der Partikelgrößenverteilung bezogen auf die Massen- oder Volumenverteilung (d(0,5)-Wert, bezogen auf in trockener Form gemessene Partikel) von 370 bis 550 $\mu$m aufweist und der Träger ein Alginat- oder Polyvinylalkohol-Träger ist.

2. Verfahren nach Anspruch 1, wobei die partikuläre Träger-immobilisierte Saccharoseisomerase-Biomasse einen Medianwert der Partikelgrößenverteilung bezogen auf die Massen- oder Volumenverteilung (d(0,5)-Wert, bezogen auf in trockener Form gemessene Partikel) von 450 bis 470 $\mu$m aufweist.

3. Verfahren nach Anspruch 1 oder 2, wobei die partikuläre Träger-immobilisierte Saccharoseisomerase-Biomasse kugelförmig ist.

4. Verfahren nach einem der vorhergehenden Ansprüche, wobei das Gewichtsverhältnis von Saccharoseisomerase-Biomasse zu Träger 10 bis 6 Teile Saccharoseisomerase-Biomasse zu 6 bis 2 Teile Träger (jeweils Trockengewicht) beträgt.

5. Verfahren nach einem der vorhergehenden Ansprüche, wobei die Isomaltulose-haltige Zusammensetzung Trehalulose enthält.

6. Verfahren nach einem der vorhergehenden Ansprüche, wobei die Saccharoseisomerase-Biomasse durch Bindung, Quervernetzung oder Einschlussimmobilisierung Träger-immobilisiert ist.

7. Verfahren nach einem der vorhergehenden Ansprüche, wobei die Saccharoseisomerase-Biomasse eine Saccharoseisomerase, eine Mikroorganismenzelle mit Saccharoseisomerase-Aktivität oder ein Zellextrakt mit Saccharoseisomerase-Aktivität ist.

**8.** Verfahren nach einem der vorhergehenden Ansprüche, wobei die Saccharoseisomerase-Biomasse aus Mikroorganismen der Gattungen Escherichia, Salmonella, Serratia, Erwinia, Enterobacter, Klebsiella, Raoultella, Pectobacterium, Pseudomonas, Azotobacter, Pantoea, Leucanea, Protaminobacter oder Bacillus sp. stammt.

**9.** Verfahren nach einem der vorhergehenden Ansprüche, wobei die Saccharoseisomerase-Biomasse aus Protaminobacter rubrum, Klebsiella sp., besonders Stamm LX3 oder Stamm NK33-98-8, Klebsiella pneumoniae, besonders Stamm 342; Enterobacter sp., besonders Stamm SZ62 oder Stamm FMB1, Erwinia tasmaniensis, besonders Stamm Et1/99; Pectobacterium atrosepticum, besonders Stamm SCRI 1043; Pectobacterium carovotum, besonders Subspezies brasiliensis, besonders Stamm PBR 1692, Azotobacter vinelandii, Leucanea leucocephalia, Erwinia rhapontici, Raoultella planticola, Pseudomonas mesoacidophila, Leuconostoc mesenteroides, Pantoea dispersa, Serratia plymuthica, Serratia marcescens oder Agrobacterium radiobacter stammt.

**10.** Verfahren nach einem der vorhergehenden Ansprüche, wobei die Verfahrensschritte a) und b) in einem Festbett oder Rührkessel durchgeführt wird.

**11.** Verfahren nach einem der vorhergehenden Ansprüche, wobei die erhaltene Isomaltulose-haltige Zusammensetzung katalytisch hydriert wird.

**12.** Verfahren zur Herstellung einer Zuckeralkohol-haltigen Zusammensetzung, wobei ein Verfahren nach einem der Ansprüche 1 bis 11, anschließend eine katalytische Hydrierung der erhaltenen Isomaltulose-haltigen Zusammensetzung durchgeführt und eine Zuckeralkohol-haltige Zusammensetzung erhalten wird.

**13.** Verfahren nach Anspruch 12, wobei die Zuckeralkohol-haltige Zusammensetzung Isomalt oder eine Isomaltvariante ist.

**Claims**

**1.** A method for producing a composition containing isomaltulose from a substrate containing sucrose, comprising the following steps:

> a) contacting the substrate containing sucrose with a particulate carrier-immobilized sucrose isomerase biomass and
> b) obtaining a composition containing isomaltulose,

**characterized in that** the carrier-immobilized sucrose isomerase biomass has a median value of the particle size distribution with regard to the mass or volume distribution (d(0,5)-value, with regard to particles measured in dry form) from 370 to 550 $\mu$m and the carrier is an alginate or polyvinylalcohol carrier.

**2.** The method according to claim 1, wherein the particulate carrier-immobilized sucrose isomerase biomass has a median value of the particle size distribution with regard to the mass or volume distribution (d(0,5)-value, with regard to particles measured in dry form) from 450 to 470 $\mu$m.

**3.** The method according to claim 1 or 2, wherein the particulate carrier-immobilized sucrose isomerase biomass is spherical.

**4.** The method according to one of the preceding claims, wherein the weight ratio of sucrose isomerase biomass to carrier is 10 to 6 parts sucrose isomerase biomass to 6 to 2 parts of the carrier (in each case dry weight).

**5.** The method according to one of the preceding claims, wherein the composition containing isomaltulose contains trehalulose.

**6.** The method according to one of the preceding claims, wherein the sucrose isomerase biomass is carrier-immobilized by bonding, crosslinking or entrapment.

**7.** The method according to one of the preceding claims, wherein the sucrose isomerase biomass is a sucrose isomerase, a microorganism cell with sucrose isomerase activity or a cell extract with sucrose isomerase activity.

8. The method according to one of the preceding claims, wherein the sucrose isomerase biomass originates from microorganisms of the genera Escherichia, Salmonella, Serratia, Erwinia, Enterobacter, Klebsiella, Raoultella, Pectobacterium, Pseudomonas, Azotobacter, Pantoea, Leucanea, Protaminobacter or Bacillus sp.

9. The method according to one of the preceding claims, wherein the sucrose isomerase biomass originates from Protaminobacter rubrum, Klebsiella sp, particularly strain LX3 or strain NK33-98-8, Klebsiella pneumoniae, especially strain 342; Enterobacter sp, particularly strain SZ62 or strain FMB1, Erwinia tasmaniensis, particularly strain Et1/99; Pectobacterium atrosepticum, particularly strain SCRI 1043; Pectobacterium carovotum, particularly subspecies brasiliensis, particularly strain PBR 1692, Azotobacter vinelandii, Leucanea leucocephalia, Erwinia rhapontici, Raoultella planticola, Pseudomonas mesoacidophila, Leuconostoc mesenteroides, Pantoea dispersa, Serratia plymuthica, Serratia marcescens or Agrobacterium radiobacter.

10. Method according to one of the preceding claims, wherein the method steps a) and b) are carried out in a fixed bed or stirred tank.

11. The method according to one of the preceding claims, wherein the obtained composition containing isomaltulose is catalytically hydrogenated.

12. The method for the production of a composition containing sugar alcohol, wherein a method according to one of claims 1 to 11 and then a catalytic hydrogenation of the resultant composition containing isomaltulose are carried out and a composition containing sugar alcohol is obtained.

13. The method of claim 13, wherein the composition containing sugar alcohol is isomalt or an isomalt variant.


**Revendications**

1. Procédé de production d'une composition contenant de l'isomaltulose à partir d'un substrat contenant du saccharose, comprenant les étapes de procédé suivantes :

   a) la mise en contact du substrat contenant du saccharose avec une biomasse de saccharose isomérase immobilisée sur un support particulaire, et
   b) l'obtention d'une composition contenant de l'isomaltulose,

   **caractérisé en ce que** la biomasse de saccharose isomérase immobilisée sur un support particulaire présente une valeur médiane de la distribution des tailles particulaires par rapport à la distribution en masse ou en volume (d(0,5), pour des particules mesurées sous forme sèche) de 370 à 550 $\mu$m et que le support est un support en alginate ou en alcool polyvinylique.

2. Procédé selon la revendication 1, dans lequel la biomasse de saccharose isomérase immobilisée sur un support particulaire présente une valeur médiane de la distribution des tailles particulaires par rapport à la distribution en masse ou en volume (d(0,5), pour des particules mesurées sous forme sèche, de 450 à 470 $\mu$m.

3. Procédé selon la revendication 1 ou la revendication 2, dans lequel la biomasse de saccharose isomérase immobilisée sur un support particulaire a une forme sphérique.

4. Procédé selon l'une des revendications précédentes, dans lequel le ratio en poids de la biomasse de saccharose isomérase par rapport au support est de 10 à 6 parties de biomasse de saccharose isomérase pour 6 à 2 parties de support (respectivement en poids à sec).

5. Procédé selon l'une des revendications précédentes, dans lequel la composition contenant de l'isomaltulose contient du tréhalulose.

6. Procédé selon l'une des revendications précédentes, dans lequel la biomasse de saccharose isomérase est immobilisée sur le support par une liaison, une réticulation ou une immobilisation par inclusion.

**7.** Procédé selon l'une des revendications précédentes, dans lequel la biomasse de saccharose isomérase est une saccharose isomérase, une cellule de microorganisme avec une activité de saccharose isomérase ou un extrait cellulaire avec une activité de saccharose isomérase.

**8.** Procédé selon l'une des revendications précédentes, dans lequel la biomasse de saccharose isomérase provient de microorganismes des espèces Escherichia, Salmonella, Serratia, Erwinia, d'entérobactéries, de Klebsiella, Raoultella, Pectobacterium, Pseudomonas, Azotobacter, Pantoea, Leucanea, Protaminobacteroder Bacillus sp.

**9.** Procédé selon l'une des revendications précédentes, dans lequel la biomasse de saccharose isomérase provient des souches Protaminobacter rubrum, Klebsiella sp., notamment la souche LX3 ou la souche NK33-98-8, Klebsiella pneumoniae, notamment la souche 342; de l'entérobactérie sp., notamment la souche SZ62 ou la souche FMB1, Erwinia tasmaniensis, en particulier la souche Et1/99; Pectobacterium atrosepticum, en particulier la souche SCRI 1043; Pectobacterium carovotum, en particulier les espèces secondaires brasiliensis, notamment la souche PBR 1692, Azotobacter vine- landii, Leucanea leucocephalia, Erwinia rhapontici, Raoultella planti- cola, Pseudomonas mesoacidophila, Leuconostoc mesenteroides, Pantoea dispersa, Serratia plymuthica, Serratia marcescens ou Agrobacterium radiobacter.

**10.** Procédé selon l'une des revendications précédentes, dans lequel les étapes de procédé a) et b) sont mises en oeuvre dans un lit solide ou un récipient agité.

**11.** Procédé selon l'une des revendications précédentes, dans lequel la composition contenant de l'isomaltulose obtenue est hydratée de manière catalytique.

**12.** Procédé de production d'une composition contenant un alcool de sucre, dans lequel un procédé selon l'une des revendications 1 à 11 est mis en oeuvre, ensuite une hydratation catalytique de la composition contenant l'isomaltulose est mise en oeuvre et une composition contenant un alcool de sucre est obtenue.

**13.** Procédé selon la revendication 12, dans lequel la composition contenant de l'alcool de sucre est de l'isomalt ou une variante d'isomalt.

# Figur 1:

## Figur 2:

**Figur 3:**

Partikelgrößenverteilung

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1424074 A1
- EP 0483755 B1
- EP 0794259 B1
- EP 0091063 A2
- EP 0625578 A1

**In der Beschreibung aufgeführte Nicht-Patentliteratur**

- **DIE OLIVA-NETO et al.** Isomaltulose production from sucrose by Protaminobacter rubrum immobilized in calcium alginate. *Bioresource Technology,* vol. 100 (18), 4252-4256
- **KAWAGUTI et al.** Production of Isomaltulose using Erwinia sp. D12 cells: Culture medium optimization and cell immobilization in alginate. *Biochemical Engineering Journal,* 2006, vol. 29 (3), 270-277